# EUROPEAN PATENT SPECIFICATION

(11) **EP 0 934 091 B1**
(45) Date of publication and mention of the grant of the patent: **04.01.2006**
(21) Application number: 97927335.6
(22) Date of filing: 08.07.1997
(51) Int. Cl.: A61M 16/16

(54) **UNIT FOR ADJUSTING HUMIDIFICATION**
EINHEIT ZUR BEFEUCHTUNGSEINSTELLUNG
UNITE DE REGLAGE DE L'HUMIDIFICATION

(30) Priority: 16.07.1996 JP 18636496
(43) Date of publication of application: 11.08.1999
(73) Proprietor: Respironics, Inc., Murrysville, PA 15668-8525 (US)
(72) Inventor: Nitta, Kazufuku, Saitama-gun, Saitama-ken 349-02 (JP)
(74) Representative: O'Connell, David Christopher
(86) International application number: PCT/IB1997/000849
(87) International publication number: WO 1998/002199

(56) References cited:
- EP-A- 0 009 543
- EP-A- 0 299 381
- EP-A- 0 413 127
- WO-A-92/07601
- FR-A- 2 250 542
- FR-A- 2 636 845
- GB-A- 2 176 405

## Description

The present invention relates to a unit for adjusting humidification and a method for the preparation of a unit for adjusting humidification.

### Prior Art

For example, GB-A-2176405 (being a patent family member of Japanese Patent Application Unexamined Publication No 62.26,076) discloses a unit for adjusting humidification which uses a tube having a peripheral wall with minute openings large enough to allow water vapor to permeate therethrough yet small enough to fail to permeate water therethrough. This humidification adjusting unit is provided with one tube having a peripheral wall with minute openings, the peripheral wall being somewhat larger in diameter, and arranged in such a way that an electrically exothermic member having an inner diameter of at least 3 mm or larger is disposed in the one tube. Upon use, the tube is allowed to be communicated to a water supply source for supplying water, and it is filled in with water always during use and water vapors are allowed to be discharged by the aid of heat generated from the electrically exothermic member from the inside of the tube to the outside of the hollow fibers through the minute openings provided in the peripheral wall.

Therefore, such a humidification adjusting unit as disclosed therein can not only ensure the function of humidifying if it is located, for example, in an intake path (an intake circuit) of an artificially respiratory device but also be disposed in an intake path by taking advantage of an outer shape of the humidification adjusting unit being in the form of a tube. When it is employed as a humidifying unit, it does not require a large volume chamber.

### Problems Sought to be Solved by The Invention

For the humidification adjusting unit as described herein above, however, there is provided one tube somewhat larger in diameter in order to ensure the generation of a certain amount of water vapors. Once the tube would be broken, an unacceptably large amount of water may be forced to leak outside the tube and such leakage will suffer from various inconvenience. For example, in the case where the such unit for adjusting humidification is employed as an intake path for an artificially respiratory device, it may become hazardous if leaked water would cause a decrease in the humidifying function and such leaked water would flow into the intake side of the artificially respiratory device, i.e. the side on which it is connected to a patient.

Given the foregoing situation, the present invention has been performed and it has the primary object to provide a unit for adjusting humidification capable of remarkably improving inconvenience that may otherwise be caused resulting from damages, even if such damages would occur, while sustaining the humidifying function and so on that are originally provided in the unit for adjusting humidification.

The present invention has a second object to provide a humidifying unit for an artificially respiratory device using the humidification adjusting unit.

Further, the present invention has a third object to provide a method for the preparation of the unit for adjusting humidification.

### Means to Solve the Problems

In order to achieve the primary object as described herein above, the present invention of claim 1 provides a unit for adjusting humidification comprising:
an exothermic member having an outer surface;
a plurality of hollow fibers disposed on the outer surface of the exothermic member, wherein each hollow fiber in the plurality of hollow fibers includes a water carrying channel defined by a peripheral wall of the hollow fiber for carrying water proximate to the outer surface of the exothermic member,
characterised in that the peripheral wall of each hollow fiber has a plurality of minute openings that are large enough to allow water vapour to pass therethrough yet small enough to prevent water from passing therethrough so that water carried in the channel of each hollow fiber does not contact the exothermic member.

According to a further aspect of the invention, there is provided an artificial respiratory system comprising:
(a) source of intake gases adapted to generate a flow of breathing gas;
(b) an intake circuit coupled to the source of intake gases so as to deliver the flow of breathing gas to the patient; and
(c) a unit for adjusting humidification unit disposed in the intake circuit, the unit for adjusting humidification being as defined in any one of claims 1 to 17.

A method of providing artificial respiration comprises:
(a) generating a flow of breathing gas;
(b) communicating the flow of breathing gas to the patient via a patient circuit;
(c) providing a humidification unit in the patient circuit, wherein the humidification unit is as defined as in any one of claims 1 to 17;
(d) delivering a flow of water to the plurality of hollow fibers (14) of the humidification unit; and
(e) heating the water in the plurality of hollow fibers via the exothermic member of the humidification unit.

According to a further aspect of the invention, there is provided a method for the preparation of a unit for adjusting humidification comprising:
providing a plurality of hollow fibers, wherein each hollow fiber in the plurality of hollow fibers is defined by a peripheral wall having minute openings large enough to allow water vapour to pass therethrough yet small enough to prevent water from passing therethrough; and
wrapping the plurality of hollow fibers around an exothermic member such that the plurality of hollow fibers are disposed on the outer surface of the exothermic member.

### Effects of The Invention

As the present invention as claimed in claim 1 comprises the multiple hollow fibers held on the outer surface of the exothermic member, each of the multiple hollow fibers having the peripheral wall with the minute openings each being large enough to allow water vapor to permeate therethrough yet small enough to fail to permeate water therethrough, it not only can be disposed in the path small in diameter but can exhibit its humidifying function to an appropriate extent by discharging water vapors into the outside of each of the multiple hollow fibers from the inside of each of the multiple hollow fibers through the minute openings provided in each of the peripheral walls thereof simply by filling each of the multiple hollow fibers in with water and heating the multiple hollow fibers with the exothermic member.

Further, as the humidification adjusting unit is structured by the multiple hollow fibers small in size as a whole and each one hollow fiber constituting the multiple hollow fibers being minute in diameter, an amount of water leaking from the hollow fibers, if leaked therefrom, can be reduced to an extremely small level, even if the hollow fibers would be damaged, broken, etc., thereby failing to reduce its humidifying function and improving it to such an extent as such water leaking therefrom causing neither inconveniences nor substantial problems

Furthermore, as the multiple hollow fibers are held on the outer surface of the exothermic member having a certain degree of physical intensity, the exothermic member may also be employed as a member for supporting the posture of the multiple hollow fibers held to the exothermic member.

From the foregoing, the present invention can provide such a humidification adjusting unit so adapted as to sustain the humidifying ability as high as the humidification adjusting unit should originally have as well as to improve inconveniences that may otherwise be caused to occur, even when it is damaged, and in addition thereto to hold the multiple hollow fibers always at its given posture even if a member for supporting the posture thereof would not be provided separately.

As the present invention : is structured in such a manner that the exothermic member is of an extendable shape, it can improve the ability for humidifying by increasing the area of the exothermic member heating the multiple hollow fibers and the area thereof holding the multiple hollow fibers as well as the ability of holding the posture of the multiple hollow fibers held to the exothermic member.

As the present invention is structured such that the exothermic member consists of the electrically exothermic member, the amount of heat to be applied to the water in the multiple hollow fibers can be controlled without waste and with a high degree of precision. Accordingly, the humidification adjusting unit according to the present invention can ensure reliability and stability in its humidifying function.

As the present invention as claimed in claim 3 provides the humidification adjusting unit having the electrically exothermic member consisting of the heating wire for generating heat upon transmission of electric current and the electrically insulating member disposed around the heating wire for ensuring electrical insulation, it can ensure safety upon use because the insulating performance can be ensured even if the electrically exothermic member is employed as the exothermic member.

The present invention as claimed in claim 5 provides the humidification adjusting unit in which the heat pipe is employed as the exothermic member. Therefore, the structure of the humidification adjusting unit according to the present invention can be simplified because it does not require to ensure electrical insulation, unlike the electrically exothermic member, at least in a region in which the multiple hollow fibers are held and humidification is performed.

In addition, the heat pipe is made of a member generally having a physical intensity so that the ability of holding the posture of the humidification adjusting unit according to the present invention can be enhanced to a higher extent by taking advantage of the intensity of the heat pipe for holding the posture of the multiple hollow fibers held on the heat pipe employed as the exothermic member.

In accordance with the present invention , as the heater is employed as a heat source for heating the exothermic section of the exothermic member, the unit for adjusting humidification according to the present invention not only can perform a secure operation of the action of the heat pipe as the exothermic member, it can also offer the same action and effects as achieved by the present invention as claimed in claim 5.

As the present invention provides the humidification adjusting unit in which the multiple hollow fibers are wound around the exothermic member, the secure contact of the multiple hollow fibers with the exothermic member can be ensured, thereby capable of improving the transmission of heat from the exothermic member to the multiple hollow fibers and the humidifying ability thereof can be readily increased or decreased by adjusting the amount of the multiple hollow fibers wound around the exothermic member.

In accordance with the present invention as claimed in claim 6, the multiple hollow fibers are wound around the exothermic member in a spiral manner so that the same action and effects as achieved by the present invention described above can also be achieved thereby.

As the present invention as claimed in claim 7 provides the humidification adjusting unit in which the multiple hollow fibers are wound around the exothermic member in such a state that the axis of each of the multiple hollow fibers is arranged so as to be generally parallel to the axis of the exothermic member, it can also perform the same action and effects as achieved by the present invention described above.

The present invention as claimed in claim 9 provides the humidification adjusting unit in which the multiple hollow fibers are bundled together at either of the sides of the exothermic member and the bundled portion of the multiple hollow fibers is formed with the connector to be connected to the side of the water supply source. Therefore, the humidification adjusting unit according to the present invention does not require to prepare for a connector separately so that it can reduce the number of parts therefor.

In addition, as the connector to which the supply water is located on the one end side of the exothermic member, the supporting of the humidification adjusting unit and the supply of water thereto can be performed at one location by mounting the one end side thereof on the mounting object, thereby capable of simplifying the manner of use of the humidification adjusting unit.

As the present invention as claimed in claim 10 provides the unit for adjusting humidification in which the multiple hollow fibers are bundled together at each of the both sides of the exothermic member and each of the bundled portions of the multiple hollow fibers is formed with the connector to be connected to the side of the water supply source, it is not necessary to prepare the connector separately, thereby capable of reducing the number of parts therefor. In addition, as water can be supplied to the multiple hollow fibers from the both end sides thereof, the supply of water to each of the multiple hollow fibers can be performed at a high speed and with high precision. In this case, as the peripheral wall of each of the multiple hollow fibers is provided with such minute openings as each being large enough to allow water vapor to permeate therethrough yet small enough to fail to permeate water therethrough, the air present in each of the multiple hollow fibers can be discharged to the outside through the minute openings formed in the peripheral wall of each of the multiple hollow fibers as the water is supplied thereto even if the water is supplied to the multiple hollow fibers from their both ends. Therefore, the humidification adjusting unit can be employed in such a state that the multiple hollow fibers are filled in with water alone without the existence of air therein.

Furthermore, even if the multiple hollow fibers on the side of the one connector would be blocked, water can be supplied from the side of the other connector. This can highly ensure the certainty of supplying water to the multiple hollow fibers.

As the present invention provides the unit for adjusting humidification in which the both sides of the multiple hollow fibers are bundled together on either side of the exothermic member and the bundled portion of the multiple hollow fibers is formed with the connector to be connected to the side of the water supply source. Hence, the present invention can achieve the same action and effects as achieved by the present invention as claimed in claim 9 and 10.

The present invention as claimed in claim 12 provides the humidification adjusting unit in which the humidifying structural body is structured by the multiple hollow fibers that are held on the outer peripheral surface of the exothermic member and that have each the peripheral wall with the minute openings each of which is large enough to allow water vapor to permeate therethrough yet small enough to fail to permeate water therethrough and in which the humidifying structural body is disposed in the short connection tube so as to be detachably connected to the tube to be connected thereto. Hence, this invention can remarkably improve inconveniences that may otherwise be caused by damages even if such damages would occur, while sustaining the humidifying ability and so on that should be originally held by the humidification adjusting unit.

Further, the humidification adjusting unit according to the present invention as claimed in claim 13 can always hold its predetermined posture without the aid of a separate unit for supporting the posture of the multiple hollow fibers. In addition, the humidification adjusting unit can be set ready for use simply by connecting the short connection tube to the tube connected thereto.

The present invention as claimed in claim 13 uses the straight tube for the short connection tube to achieve the same action and effects as achieved by the present invention as claimed in claim 12 in the manner as described herein above.

As the present invention as claimed in claim 14 provides the humidification adjusting unit having the mounting flange mounted detachably on the short connection tube, thereby simplifying the mounting of the humidifying structural body on the short connection tube as well as achieving the same action and effects as achieved by the present invention as claimed in claim 12 in the manner as described herein above.

The present invention as claimed in claim 15 uses the generally L-shaped tube for the short connection tube to achieve the same action and effects as achieved by the present invention as claimed in claim 12 in the manner as described herein above. In addition, the tube portion on the one side of the L-shaped tube can be employed as a portion for supporting (mounting) the humidifying structural body with high precision.

Furthermore, the humidifying structural body can be set ready for use simply by inserting the humidifying structural body into the mounting opening of the tube portion on the one side of the humidifying structural body. This can improve mounting operability.

The present invention as claimed in claim 16 is arranged so as to readily connect the short connection tube to an object of an inner-outer double tubes structure so that it can offer the advantage that the unit for adjusting humidification can readily be set to such an object as well as it can achieve the action and effects similar to those as achieved by the present invention as claimed in claim 15.

The present invention can accommodate the humidifying structural body in its entirety within the short connection tube and therefore protect the humidifying structural body by the short connection tube as well as achieve the action and effects similar to those of the present invention as claimed in claim 15 in the manner as described herein above.

The present invention as claimed in claim 18 can achieve the action and effects similar to those as achieved by each of the present invention as claimed in claims 1 to 17, inclusive, even when the unit for adjusting humidification is disposed in an intake circuit of an artificially respiratory device for the humidification of intake gases.

As the present invention as claimed in claim 18 can humidify intake gases in the intake circuit using the humidification adjusting unit, there can be provided a humidifying unit for an artificial respiratory device using the humidification adjusting unit.

### Manners of Practicing The Invention

The present invention will be described by way of examples with reference to the accompanying drawings.

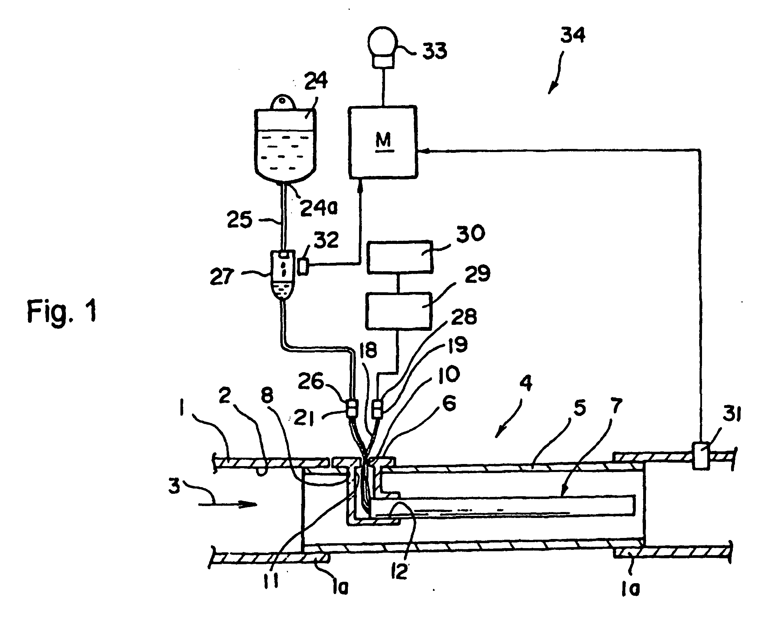

Figs. 1 to 7 show the humidification adjusting unit according to the first embodiment for practicing the present invention. As shown in Fig. 1, reference numeral 1 denotes an intake tube of an intake circuit in an artificially respiratory device connecting an adapter (not shown) connected to the lung of a patient to a source of supplying intake gases, although not shown, an inner portion of the intake tube 1 constituting an intake path 2. Intake gases 3 (the existence of intake gases and the direction of their flow being indicated by the arrow) flow toward the adapter from the source of supplying the intake gases. The inner diameter of the intake path 2 may be set to be in the range of, for example, from 14 mm to 16 mm for an infant patient and from 20 mm to 24 mm for a grown-up patient.

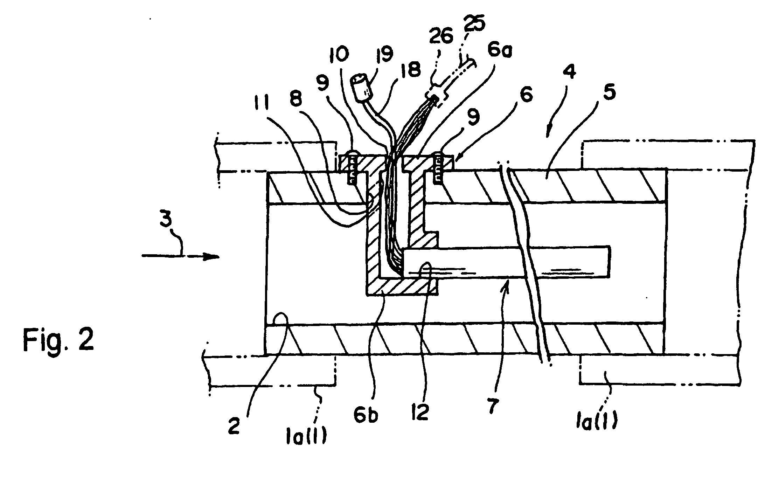

As shown in Figs. 1 and 2, in the embodiment according to the present invention, the intake tube 1 is mounted detachably on a humidification adjusting unit 4 that is provided with a short connection tube 5, a mounting flange 6, and a humidifying structural body 7.

The short connection tube 5 may be a straight tube and its both end portions are engaged (connected) in an airtight fashion, each being connected to the connection end section of the connection end portion la of the intake tube 1 to be connected thereto. The outer diameter of the short connection tube 5 is set so as to be somewhat larger than the inner diameter of the intake tube 1, in order to ensure the relationship of airtight engagement with the intake tube I to be connected thereto, and it may be set appropriately in accordance with the intake tube I to be employed. As a matter of course, in this case, the relationship of engagement of the short connection tube 5 with the connection end portion la of the intake tube 1 may be such that the inner periphery of the short connection tube 5 is engaged with the outer periphery of the connection end portion la of the intake tube 1. In such a case, the inner diameter of the short connection tube 5 is set to be somewhat smaller than the outer diameter of the connection end portion la of the intake tube 1 to be connected thereto, in order to ensure the relationship of airtight engagement with the intake tube 1 to be connected thereto. Further, in this case, it is needless to say that the use of a packing, a fastening band or the like may be preferably employed in order to enhance airtightness.

The peripheral wall on the one end side of the short connection tube 5 (on the left side in Figs. 1 and 2) is formed with a mounting opening 8 for mounting the mounting flange 6. The mounting opening 8 is located at the position outside of the connection end portion la of the intake tube 1 upon connection with the connection end portion 1a thereof and disposed so as to allow the inside of the short connection tube 5 to be communicated with the outside thereof.

The mounting flange 6 consists of a flange portion 6a in a square-plate shape or the like and a cylindrical holding portion 6b.

The flange portion 6a of the mounting flange 6 is shaped so as to be disposed along an outer peripheral wall of the short connection tube 5 and it is fixed to the short connection tube 5 by a screw 9 so as to cover the mounting opening 8 of the short connection tube 5 in a tight manner. The flange portion 6a is further provided with a communicating opening 10 that allows the mounting opening 8 of the short connection tube 5 to open to the outside in the center of the flange portion 6a.

The holding portion 6b of the mounting flange 6 is disposed so as to stand upright with respect to the plate surface of the flange portion 6a in such a state in which the communicating opening 10 of the flange portion 6a faces an opening 11 at the base end thereof. The holding portion 6b is arranged to be inserted through the mounting opening 8 of the short connection tube 5 over the entire length of the mounting opening 8 of the short connection tube 5, extending up to the center of the short connection tube 5 in the radial direction, and then curved on the other end side of the short connection tube 5 at a generally right angle (on the right end side in Figs. 1 and 2). The end opening 12 is disposed so as to face the opening on the other end of the short connection tube 5. The end opening 12 at the one end of the holding portion 6b is large enough in size to be engaged with and held with the humidifying structural body 7 and the end opening 12 is arranged so as to be communicated to the outside through the communicating opening 10 of the flange portion 6a in the holding portion 6b.

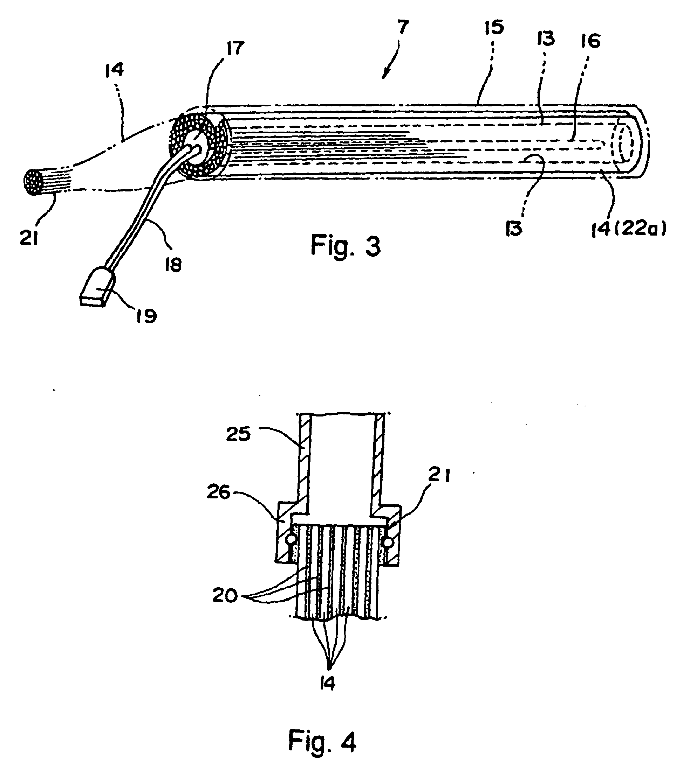

As shown in Fig. 3, the humidifying structural body 7 comprises the exothermic member 13, the multiple hollow fibers 14 to be held at the outer surface of the exothermic member 13, and the mesh cover tube 15 to be inserted into and engaged with the exothermic member 13 and the multiple hollow fibers 14.

In this embodiment of the present invention, the exothermic member 13 is an electrically exothermic member of a columnar and extendable shape and may have a diameter in the range of, for example, from approximately 3 mm to 6 mm and an entire length in the range of, for example, from approximately 1 m to 2 m. The length in the extendable direction (the length in left-hand and right-hand directions in Figs. 1 and 2) of the exothermic member 13 is set to be shorter than the axial length of the short connection tube 5. The exothermic member 13 comprises a heating wire 16 (a heater wire) and an electrically insulating member 17 enclosing the periphery of the heating wire 16, in order to ensure an electrical configuration. The heating wire 16 is disposed in the inside of the electrically insulating member 17 and has the function of generating heat upon supplying electric current. A connection code 18 in a covering state to be connected to the heating wire 16 extends toward the outside from the one end side of the exothermic member 13. The top end portion of the connection code 18 is provided with one of connectors 19 for supplying electric current. The insulating member 17 ensures insulating performance toward the outside and both of the insulating member 17 and the heating wire 16 ensure somewhat high intensity.

Each of the multiple hollow fibers 14 constituting the multiple hollow fibers 14 has the peripheral wall with the minute openings (for example, a porosity rate of approximately 57.8%) each of which allows water vapor to permeate therethrough yet fails to permeate water therethrough. Each of the multiple hollow fibers is minute in size (for example, an outer diameter, 413 microns; an inner diameter, 282 microns; an entire length, approximately 1.2 meters - 1.5 meters). In the embodiment of the present invention, the multiple hollow fibers (the total number of from 40 to 60 hollow fibers in this embodiment) 14 are disposed in a state such that the axis of each of the multiple hollow fibers 14 is arranged about its entire periphery so as to be nearly parallel to the axis of the exothermic member 13 and the entire periphery of the exothermic member 13 is wound with the multiple hollow fibers 14. In this case, the multiple hollow fibers 14 are folded in the middle in the direction in which they extend and the exothermic member 13 is wound with the folded multiple hollow fibers 14. The end portion on the path opening side of the both end portions of each of the multiple hollow fibers 14 is arranged so as to extend outward from the one end side of the exothermic member 13 in a state in which the ends of the multiple hollow fibers are arranged even.

It is now provided that the folded portion of the multiple hollow fibers 14 is rounded so as not to be squashed.

As shown in Fig. 3 and 4, the both end portions of each of the multiple hollow fibers 14 in such an even arranged state are joined together at a position outside of either of the one end sides of the exothermic member 13 with an adhesive agent 20 into a bundled state. The bundled portion is formed with a one connector 21 of the connectors in a given shape for supplying water. Upon the formation of the one connector 21, the adhesive agent 20 may penetrate into the inside of the multiple hollow fibers 14 so that the one end portion of the bundled multiple hollow fibers 14 into which the adhesive agent has penetrated is cut in round slices to form a new end surface. The connection code 18 is disposed so as to extend toward the outside through the multiple hollow fibers 14.

As shown in Fig. 3, the mesh cover tube 15 is in a mesh form extendable in both of its axial and radial directions. The mesh cover tube 15 has an opening extending axially over the entire length and communicating the inside to the outside thereof. The exothermic member 13 and the multiple hollow fibers 14 wound around the outer surface of the exothermic member 13 are inserted into the mesh cover tube 15 in a tight fashion. The multiple hollow fibers 14 are allowed to press the outer surface of the exothermic member 13 by an appropriate degree of pressing force that is created by the force upon the contraction of the mesh cover tube 13.

The humidifying structural body 7 is mainly of an extendable shape depending upon the shape of the exothermic member 13 and it is shaped such that the diameter of the humidifying structural body 7 is smaller than the inner diameter of the short connection tube 5 and that the length in its extending direction is shorter than the axial length of the short connection tube 5. The humidifying structural body 7 is arranged so as to be accommodated in its entirety within the short connection tube 5.

A one end portion (the left end portion in Figs. 1 and 2) of the humidifying structural body 7 is held in a tight engagement with the end portion of the holding portion 6b of the mounting flange 6 in the short connection tube 5. On the other hand, the other end portion (the right end portion in Figs. 1 and 2) of the humidifying structural body 7 is a free end side extending toward the other end side of the short connection tube 5. Further, the connection code 18 and the multiple hollow fibers 14 are extended in the holding portion 6b toward the outside of the mounting flange 6 through the communicating opening 10. The one connector 21 in the connection code 18 and the one connector 19 for supplying electric current in the multiple hollow fibers 14 are located outside of the mounting flange 6.

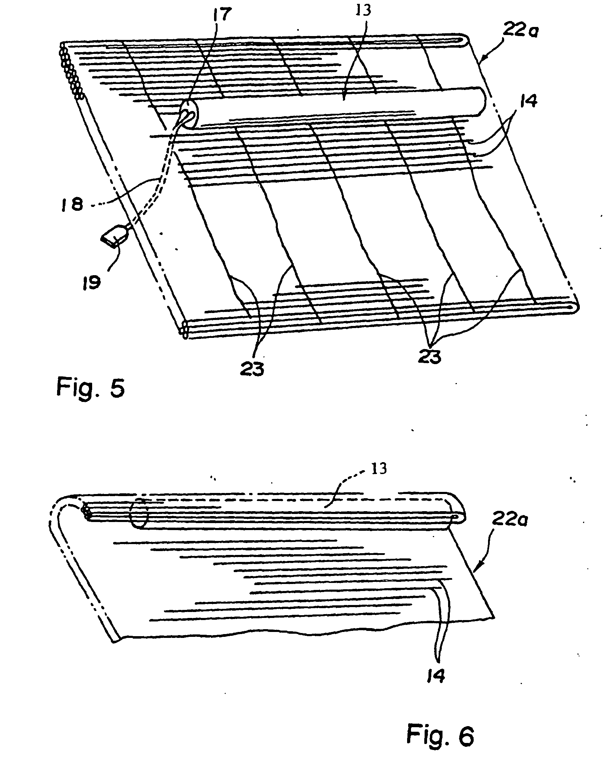

The humidifying structural body 7 may be prepared in the steps as shown in Figs. 5 to 7.

As shown in Fig. 5, first, the multiple hollow fibers 14 each having the peripheral wall with the minute openings each of which is large enough to allow water vapor to permeate therethrough yet small enough to permeate water therethrough are prepared so as to be formed into a multiple hollow fibers gathering 22a in a sheet shape in which the axis of each of the multiple hollow fibers 14 is arranged so as to be generally parallel to each other. By combining the multiple hollow fibers 14 in the manner as described herein above, the multiple hollow fibers 14 can be treated in such a collective manner as failing to be scattered, whereby the work for holding the multiple hollow fibers 14 to the exothermic member 13 can be improved greatly.

In this case, a string member 23 or the like may be employed in order to join the multiple hollow fibers 14 integrally to each other into the multiple hollow fibers gathering.

Then, as shown in Fig. S, the multiple hollow fibers gathering 22a is folded in the middle in the direction in which each of the multiple hollow fibers 14 extends, and the exothermic member 13 is disposed on the folded multiple hollow fibers gathering 22a. At this time, the other end portion of the exothermic member 13 is arranged so as to become even with the folded portion of the multiple hollow fibers gathering 22a, while attention is being paid to the fact that the folded portion of the multiple hollow fibers gathering 22a is not squashed and that the axis of the exothermic member 13 is arranged so as to be generally parallel to the axis of each of the multiple hollow fibers 14. Further, the both end portions of the multiple hollow fibers 14 are disposed so as to project outward from the one end portion of the exothermic member 13.

Thereafter, as shown in Fig. 6, the folded multiple hollow fibers gathering 22a is wound around the exothermic member 13 while sustaining the state in the manner as described herein above. By holding the multiple hollow fibers 14 in that state to the outer surface of the exothermic member 13 and arranging the multiple hollow fibers 14 so as to increase the area of the exothermic member 13 in which the multiple hollow fibers 14 are held to the exothermic member 13, the humidifying ability of the humidifying structural body 7 (the humidification adjusting unit 4) is improved.

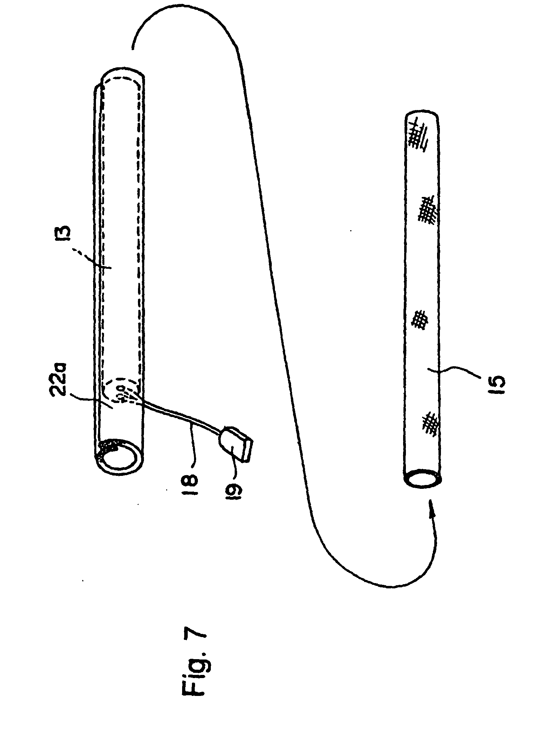

At this time, as shown in Figs. 5 and 7, the connection code 18 is disposed extending through the multiple hollow fibers 14 and projecting toward the outside of the wound multiple hollow fibers gathering 22a. This can easily conduct the work for forming the one connector 21 easy to be conducted in the step that follows

Then, as shown in Fig. 7, the multiple hollow fibers gathering 22a and the exothermic member 13 are inserted tightly into the extendable mesh cover tube 15. The multiple hollow fibers 14 are held with high precision to the exothermic member 13 and each of the multiple hollow fibers 14 is in contact with the exothermic member 13 with an appropriate degree of the pressing force, thereby improving the transmission of heat from the exothermic member 13 to each of the multiple hollow fibers 14.

Next, as shown in Figs. 3 and 4, the end portion of the folded hollow fibers gathering 22a on its path opening side (the both end portions of the multiple hollow fibers 14) is joined together with the adhesive agent 20 into a bundle. The bundled portion is formed with the one connector 21 to form the humidifying structural body 7. This does not require a connector to be prepared separately and therefore can reduce the number of parts. Further, this allows water to be supplied to the multiple hollow fibers 14 from the both end sides of the multiple hollow fibers 14, for example, thereby capable of filling each of the multiple hollow fibers 14 in with water at a high speed and with a high degree of precision.

At this time, the one connector 21 may be formed by combining the both end portions of the multiple hollow fibers 14 together or by separately combining each of the end portions of the multiple hollow fibers 14 together.

As shown in Fig. 1, the one connector 21 of the humidifying structural body 7 prepared in the manner as described herein above is connected through a water supply tube 25 to a water bag 24 as a source of supplying water. The water bag 24 is filled with a predetermined amount of water and the amount of the water filled in the water bag 24 can be visually confirmed from the outside because the water bag 24 is made of a transparent or translucent material. The water bag 24 is mounted on a pole (not shown) in a hung state and the water filled in the water bag 24 is provided with a predetermined position head. The water bag 24 is further provided at its lower portion with a water outlet 24a and at its upper portion with an opening (not shown) through which the inside of the bag communicates to the outside.

The water supply tube 25 has its one end portion connected (for example, held by engagement) to the water outlet 24a of the water bag 24 and its other end portion formed with the other connector 26 (schematically shown) as the counterpart of the one connector 21, as shown in Fig. 1 and 4. The other connector 26 is formed so as to be engaged with the one connector 21 in an airtight manner by a one-touch operation.

Further, as shown in Fig. 1, the water supply tube 25 is provided with a water drops count device 27 as a flow meter for measuring the flow of water. The water drops count device 27 is so arranged as to allow a flow of water on the side of the connector 21 formed in the multiple hollow fibers 14 after the water has been dropped from the water bag 24 into the water drops count device 27. Further, the water drops count device 27 is so arranged as to visually confirm the state in which the water drops from the outside.

To the one connector 19 for the supply of electric current, as shown in Fig. 1, is connected the other connector 28 for the supply of electric current, and the other electric current supply connector 28 in turn is connected to a power source 30 through an adjustment unit 29. By connecting both of the electric current supply connectors 19 and 28, electric current is allowed to be supplied to the heating wire 16 generating heat and thus warming each of the multiple hollow fibers 14 to discharge water vapors into intake gases 3 from the multiple hollow fibers 14. The amount of the water vapors to be discharged at this time may be adjusted automatically on the basis of the difference between the pressure of the water vapors in the multiple hollow fibers 14 and the pressure of the water vapors in the intake path 2.

It is to be provided, however, that the temperature of the intake gases is set by the electrically exothermic member 13 to fail to become higher than a predetermined degree of the temperature, that is, the temperature at which inconveniences may be caused to occur for the patient.

In Fig. 1, reference symbol M denotes a monitor. The monitor M is supplied on the downstream side of the humidification adjusting unit 4 with signals from a temperature-moisture sensor 31 for sensing the temperature and the moisture of the intake gases 3 and signals from a water drops detecting sensor for sensing water drops in the water dropping device 27. The monitor M monitors the contents of these signals and operates an alarm means (for example, an alarm lamp or an alarm buzzer) as it detects them as being higher than the set level.

Therefore, the humidifying unit 34 with the structure as described herein above, as a matter of course, can demonstrate the humidifying ability for humidifying the intake gases 3 to an appropriate extent on the basis of the discharging of water vapors from the humidifying structural body 7 when the humidifying structural body 7 is disposed in the intake path 2 having a small diameter. Further, the humidifying unit 34 is so adapted as to monitor the breakage of the hollow fibers 14 or other damages (i.e. a leakage of water from the hollow fibers 14) with extremely high precision because the water drops detecting sensor can sense the leakage of water, even if very small, from the hollow fibers 14 due to the breakage or damages of the hollow fibers 14 as a variation in the state in which water drops on the side of the water dropping unit 27 (i.e. whether the set state is satisfied), and the state detected is transmitted to the monitor M and then to the alarm means 33.

Furthermore, in the embodiment of the present invention, in addition to the contents as described herein above, the multiple hollow fibers 14 are structured by the multiple hollow fibers 14 having a small size in the entirety and each of the multiple hollow fibers 14 constituting the multiple hollow fibers 14 is extremely small in diameter. Therefore, even if the hollow fibers 14 would be broken or damaged or undergo other faults, the amount of water leaking from the hollow fibers 14 can be controlled to an extremely small level so that the humidifying unit according to the present invention does not reduce the humidifying ability and can improve the inconveniences and problems which may otherwise be caused due to the leakage of water to such an extent as causing any such inconveniences and problems to no substantial level, even if such would be caused to occur.

In this case, although the multiple hollow fibers 14 are generally flexible, they are held on the exothermic member 13 that is physically stronger to some extent so that the exothermic member 13 can demonstrate the function of supporting the flexible hollow fibers 14. Therefore, the multiple hollow fibers 14 can always sustain their predetermined posture without the disposition of a posture supporting means separately by taking advantage of the exothermic member 13 as a unit for holding the posture of the multiple hollow fibers 14, too.

Further, the exothermic member 13 is of a columnar shape extendable in both directions so that the area of the exothermic member for heating the multiple hollow fibers 14 and the area thereof for holding them can be increased readily, thereby enabling improvements in the humidifying ability and enhancing the ability of holding the posture of the multiple hollow fibers 14 to a higher extent.

Furthermore, as the exothermic member 13 consists of the electrically exothermic member, the amount of heating the multiple hollow fibers 14 (i.e. the water contained therein) can be controlled without waste and with high precision, thereby providing reliability and safety for the humidifying ability of the humidification adjusting unit 4 according to the present invention.

In this case, there is provided the electrically insulating member 17 enclosing the heating wire 16 for ensuring insulation. Thus, even if the electrically exothermic member is employed as the exothermic member 13, insulation against the outside can be ensured and at the same time safety upon use can be ensured.

Furthermore, the multiple hollow fibers 14 are wound around the exothermic member 13 in such a manner that the axis of each of the multiple hollow fibers 14 is arranged so as to be generally parallel to the axis of the exothermic member 13, thereby ensuring a secure contact of each of the multiple hollow fibers 14 with the outer surface of the exothermic member 13 and improving the transmission of heat to each of the multiple hollow fibers 14 from the exothermic member 13. In addition, the amount of the multiple hollow fibers 14 wound around the exothermic member 13 can be readily adjusted, thereby allowing a ready adjustment of an increase or a decrease of the humidifying ability of the humidification adjusting unit according to the present invention.

In addition, the multiple hollow fibers 14 are bundled outside the one end side of the exothermic member 13 and the bundled portion is formed with the one connector 21 to be connected to the water bag 24. Therefore, the humidification adjusting unit according to the present invention can reduce the number of parts because such a connector is not required to be prepared separately.

Further, the one connector 21 for supplying water is located on the one end side of the exothermic member 13. By using the one end side of the exothermic member 13 as the side for supporting a mounting object, the supporting system, the water supply system and the electric current supply system of the humidification adjusting unit 4 according to the present invention can be concentrated at one location, thereby capable of simplifying the manners of use and the structure of connection and making the connection work more efficiently.

At this time, each of the multiple hollow fibers 14 can be filled in with water at a high speed and with high precision by supplying water to the multiple hollow fibers 14 from their both end sides. As the peripheral wall of each of the multiple hollow fibers 14 is provided with the minute openings that are each large enough to allow water vapor to permeate therethrough yet small enough to fail to permeate water therethrough, air present in each of the multiple hollow fibers 14 can be discharged into the outside through the minute openings formed in the peripheral walls of the multiple hollow fibers as the water is supplied therein to, even if each of the multiple hollow fibers 14 is supplied with water from its both side ends. Hence, each of the multiple hollow fibers 14 can always be filled in with water alone without the presence of air therein.

Furthermore, even if the multiple hollow fibers 14 would be blocked on the connector side on the one end side of the multiple hollow fibers 14, water can be supplied to the multiple hollow fibers 14 from the connector side on the other end side of the multiple hollow fibers 14. This arrangement can enhance reliability in the water supply to each of the multiple hollow fibers 14.

Further, in the embodiment of the present invention, the humidification adjusting unit 4 is provided with the short connection tube 5 of a straight tube shape so that it can be readily set simply by connecting the short connection tube 5 to the connection end portion la of the intake tube 1. This can improve workability of setting the humidification adjusting unit.

In this case, as the humidifying structural body 7 is combined in a unit, the assembly of the humidifying structural body 7 with the short connection tube 5 can be performed in a simplified manner by using the mounting flange 6. Further, the humidifying structural body 7 can be assembled directly with the intake tube 1 in a simplified manner by forming the mounting opening 8 in the intake tube 1.

Further, as the humidifying structural body 7 is accommodated in the short connection tube 5 in its entirety, the humidifying structural body 7 is protected by the short connection tube 5, whereby the mounting operation, the transferring operation and other operations can be carried out in a convenient manner.

In addition, as the exothermic member 13 and the multiple hollow fibers 14 are inserted into the extendable mesh cover tube 15 in a tightly engaged manner, the multiple hollow fibers 14 can be held around the exothermic member 13 with high precision and the transmission of heat from the exothermic member 13 to each of the multiple hollow fibers 14 can be improved by allowing each of the multiple hollow fibers 14 to come into contact with the exothermic member 13 with an appropriate degree of pressing force.

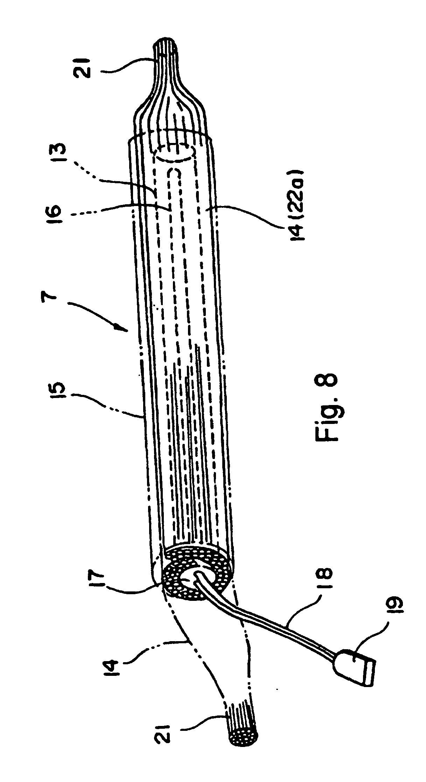

Figs. 8 et *seq.* show other embodiments of the humidification adjusting unit according to the present invention. In each of the embodiments that follow, the same structuring elements as in the first embodiment are provided with the identical reference numerals and symbols and a duplicate description will be omitted.

Fig. 8 shows the humidification adjusting unit according to the second embodiment of the present invention. In the second embodiment, the multiple hollow fibers 14 are bundled at the both end sides of the exothermic member 13 and the bundled portions are formed each with the one connector 21 to be connected to the side of the water bag 24.

In this case, the humidification adjusting unit according to this embodiment can reduce the number of parts because a connector is not required to be prepared separately. Further, water can be filled in each of the multiple hollow fibers 14 at a high speed and with high precision even if the water supply to each of the multiple hollow fibers 14 would be performed from the both end sides thereof. In addition, even if the multiple hollow fibers 14 would be blocked at the connector side outside either one side of the exothermic member 13, water can be supplied to each of the multiple hollow fibers from the connector outside the other end side of the exothermic member 13, thereby enhancing reliability in supplying water to each of the multiple hollow fibers 14.

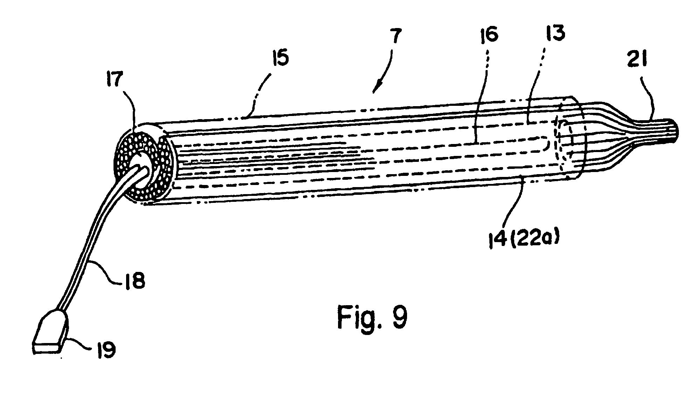

Fig. 9 shows the humidification adjusting unit according to a third embodiment of the present invention. In the third embodiment, the multiple hollow fibers 14 are bundled on the other end side of the exothermic member 13 opposite to the electric current supply side of the exothermic member 13 and the bundled portion is formed with the one connector 21 to be connected to the side of the water bag 24.

This arrangement can form the one connector 21 without taking an electric current supply system into consideration, thereby making the formation of the one connector 21 easier.

On the contrary to the case in the third embodiment, as a matter of course, the one connector 21 may be formed on the one end side of the exothermic member 13 which is the electric current supply side of the exothermic member 13.

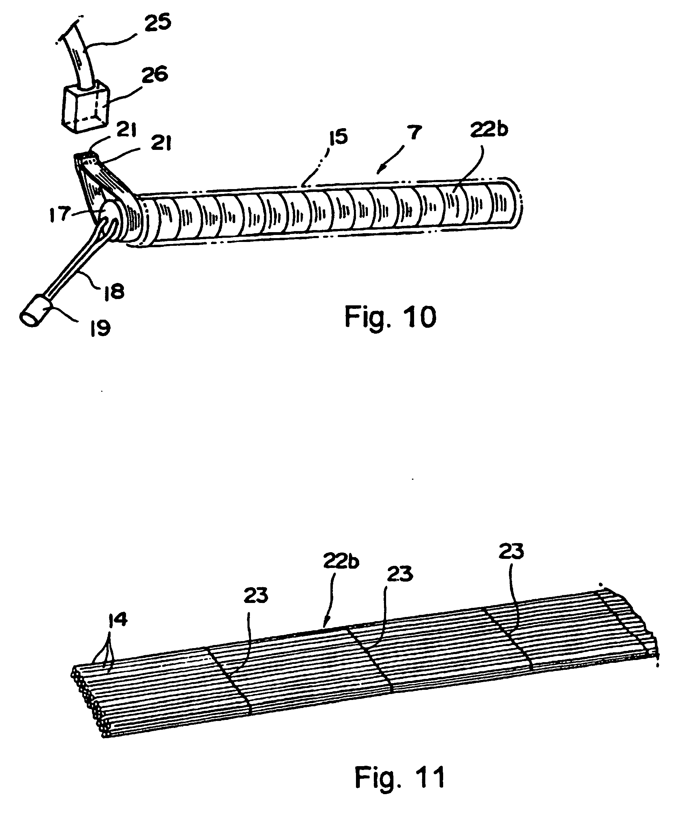

Figs. 10-12 show the humidification adjusting unit according to a fourth embodiment of the present invention. In the fourth embodiment, the multiple hollow fibers 14 each being large enough to allow water vapor to permeate therethrough yet small enough to fail to permeate water therethrough are united into the multiple hollow fibers gathering 22b formed in a tape shape by connecting each of the multiple hollow fibers 14 integrally to each other with a string member 23 or the like so as to allow the axis of each of the multiple hollow fibers 14 to become generally parallel to the axis of the exothermic member 13 (as referred to in Fig. 11). The multiple hollow fibers gathering 22b in a tape form is then wound around the exothermic member 13 in a spiral manner, and the humidifying structural body 7 is then prepared by inserting the multiple hollow fibers gathering 22b and the exothermic member 13 into the extendable mesh cover tube 15 in a tightly engaged manner.

In the preparation of the humidifying structural body 7 in the manner as described herein above, there can also be achieved the action and effects similar to those as achieved by the method for the preparation of the humidification adjusting unit in the first embodiment of the present invention.

In the fourth embodiment of the present invention, as shown in Fig. 10, the multiple hollow fibers gathering 22b in the tape form is wound around the exothermic member 13 starting with a one end portion of the exothermic member 13 over the entire length thereof to the other end portion thereof and then continually turning from the other end portion thereof back to the one end portion thereof The winding of the multiple hollow fibers gathering 22b around the exothermic member 13 in the manner as described herein above locates the both end portions of the multiple hollow fibers gathering 22b at the one end portion of the exothermic member 13 and the both end portions of the multiple hollow fibers gathering 22b are formed each with the one connector 21. As a matter of course, at this time, the both end portions of the multiple hollow fibers gathering 22b can be bundled together integrally and the bundled portion may be formed with the one connector 21.

In the fourth embodiment of the present invention, too, the amount of the multiple hollow fibers 14 held on the exothermic member 13 can be increased improving the humidifying ability. At the same time, as the both end portions of the multiple hollow fibers gathering 22b are located at the one end portion of the exothermic member 13, the humidification adjusting unit according to the fourth embodiment of the present invention can achieve the action and effects equivalent of those as achieved in the first embodiment (Fig. 3).

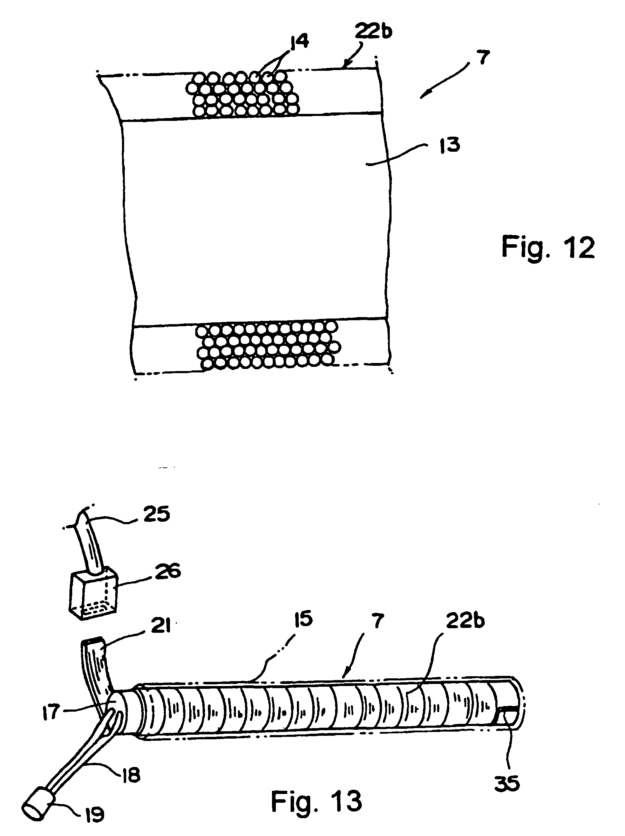

Fig. 13 shows the humidification adjusting unit in a fifth embodiment of the present invention and specifically shows a variant of the manner of winding the multiple hollow fibers gathering 22b in the tape form around the exothermic member 13 as in the fourth embodiment. In the fifth embodiment, the multiple hollow fibers gathering 22b in the tape form is wound around the exothermic member 13 starting with a one end portion of the exothermic member 13 and extending over the entire length up to the other end portion of the exothermic member 13 in such a manner that the one end portion of the multiple hollow fibers gathering 22b in the tape form is located at the one end portion of the exothermic member 13 and the other end portion of the multiple hollow fibers gathering 22b in the tape form is located at the other end portion of the exothermic member 13. Further, the openings at the other end portion 35 of the multiple hollow fibers gathering 22b in the tape form are closed with adhesive agent or the like (the state in which the openings at the other end portion 35 of the multiple hollow fibers gathering 22b in the tape form are closed is shown by oblique lines in the drawing). Furthermore, the one end portion of the multiple hollow fibers gathering 22b in the tape form is bundled and the bundled portion is formed with the one connector 21.

As the multiple hollow fibers gathering 22b in the tape form is wound around the exothermic member 13 in a spiral form in the manner as described herein above, even where the one connector 21 is to be located at the one end side of the exothermic member 13 that is the electric current supply side of the exothermic member 13, the one connector 21 can be formed without taking the electric current supply system into consideration, thereby facilitating the preparation of the one connector 21.

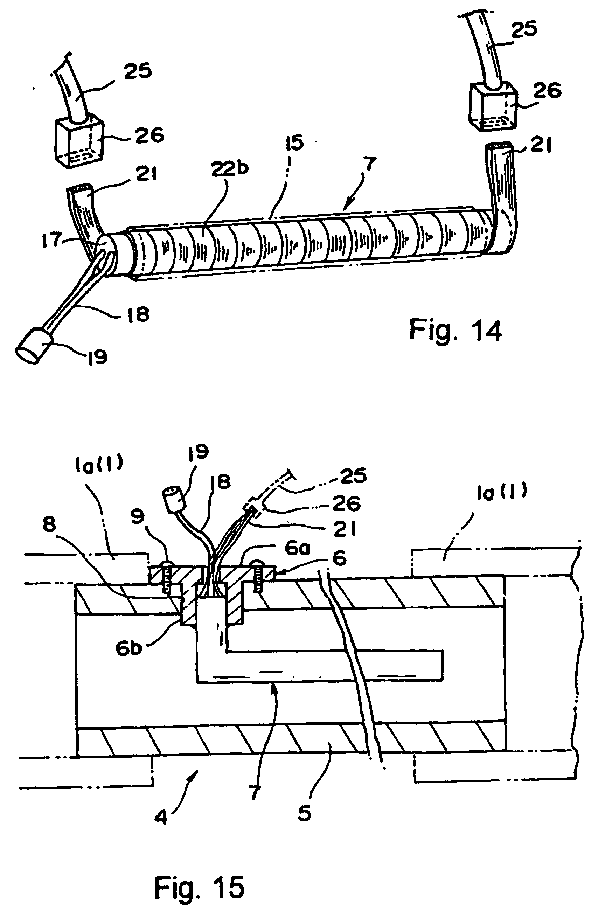

Fig. 14 shows the humidification adjusting unit in accordance with a sixth embodiment of the present invention, which is a variant of the fifth embodiment as described herein above. In the sixth embodiment, the one end portion of the multiple hollow fibers gathering 22b in the tape form wound around the exothermic member 13 is located at the one end portion of the exothermic member 13 and the other end portion of the multiple hollow fibers gathering 22b in the tape form is located at the other end portion of the exothermic member 13.

Further, the multiple hollow fibers gathering 22b in the tape form is bundled at each of the both end portions thereof and each of the bundled portions thereof is formed with the one connector 21.

This arrangement can achieve the action and effects equivalent of those as achieved by the humidifying structural body 7 in the second embodiment.

Fig. 15 shows the humidification adjusting unit in a seventh embodiment of the present invention, which indicates a variant of the humidification adjusting unit 4. In the humidification adjusting unit 4, the mounting flange 6 is arranged in such a manner that the holding portion 6b is disposed standing upright from the flange portion 6a and a one end portion of the humidifying structural body 7 is curved at a nearly right angle with respect to the other end portion thereof Further, the one end portion of the humidifying structural body 7 is held to the holding portion 6b by engagement.

In this case, too, the humidifying structural body 7 can be disposed with the other end portion thereof extending in the axial direction of the short connection tube 5 by mounting the mounting flange 6 on the short connection tube 5.

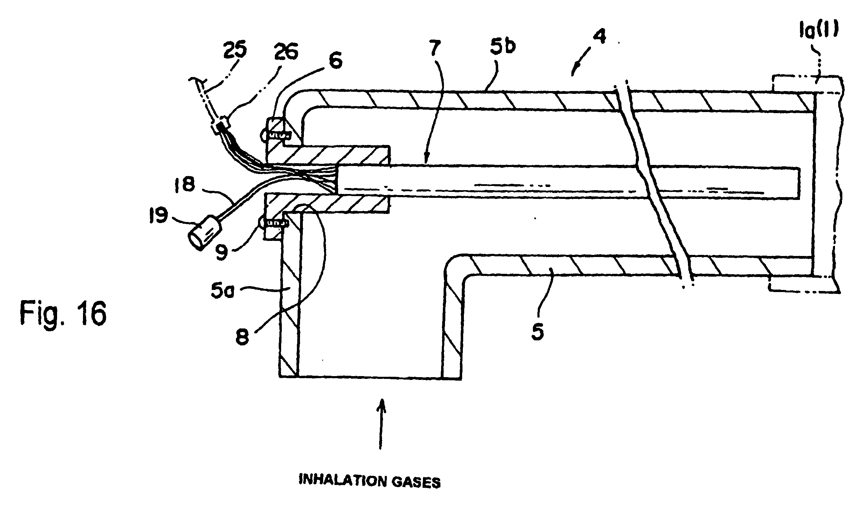

Fig. 16 shows the humidification adjusting unit in accordance with an eighth embodiment of the present invention, which indicates a variant of the humidification adjusting unit. In the eighth embodiment, the short connection tube 5 is a generally L-shaped tube and it is disposed in such a manner that a one end portion of the humidifying structural body 7 is mounted through the mounting flange 6 on the tube portion 5a on a one end side of the short connection tube 5 in a region where the one end portion of the humidifying structural body 7 faces a sectional surface of the path of the tube portion 5b on the other side of the short connection tube 5, and the other end side of the humidifying structural body 7 extends in the axial direction of the tube portion 5b on the other side of the short connection tube 5 in the tube portion 5b on the other side thereof.

This arrangement allows the generally L-shaped tube to be detachably mounted on the intake tube 1 in the same manner as in the first embodiment as described herein above. In this embodiment, the tube portion 5a on the one side of the generally L-shaped tube 5 can be employed as a supporting portion (a mounting portion) for supporting the humidifying structural body 7 with high precision and further the humidifying structural body 7 is set ready for use simply by inserting the humidifying structural body 7 into the mounting opening 8 of the short connection tube 5 at a right angle to the tube portion 5a on the one side of the short connection tube 5.

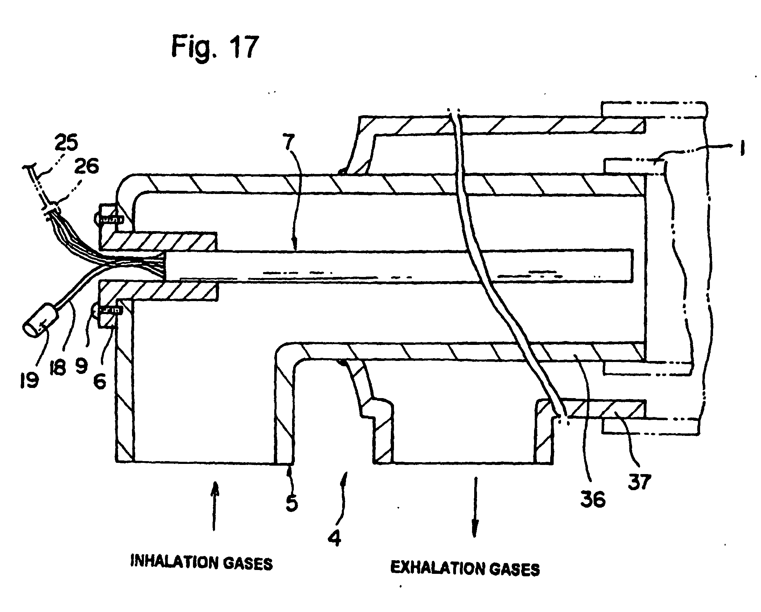

Fig. 17 shows the humidification adjusting unit according to a ninth embodiment of the present invention, which indicates a variant of the humidification adjusting unit according to the eighth embodiment as described herein above. In the ninth embodiment, the short connection tube 5 is of an inner-outer double tube structure and the tube portion 5b on the other side of the short connection tube 5 as used in the eighth embodiment may be employed as an inner tube 36 of the short connection tube according to this embodiment, and the inner tube 36 is disposed inserting an outer tube 37 so as to enclose the outer periphery of the inner tube 36, thereby forming a ring-shaped path around and between the outer wall surface of the inner tube and the inner wall surface of the outer tube.

In this embodiment, the short connection tube 5 can be connected to an artificially respiratory circuit of an inner-outer double tube structure and the humidification adjusting unit according to this embodiment can be set readily to such an artificially respiratory circuit.

In this case, exhaled gases are allowed to flow on the side of the outer tube (between the inner tube and the outer tube) and, in this case, it is possible to prevent water vapors within the inner tube from condensing using the heat from the exhaled gases flowing in the ring-shaped path.

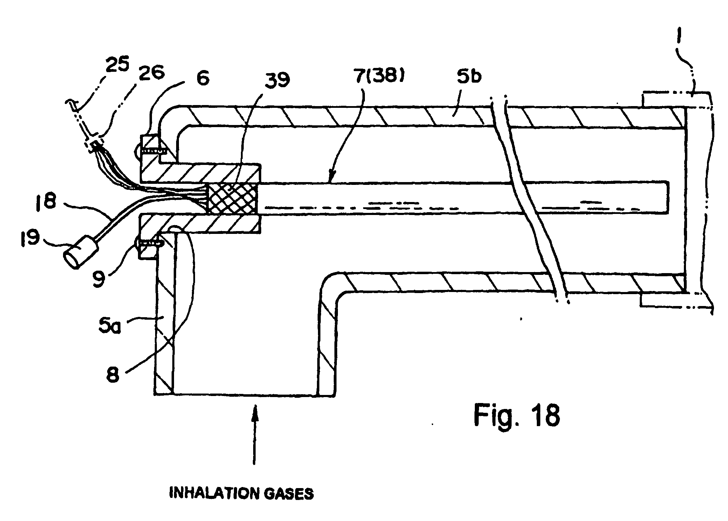

Fig. 18 shows the humidification adjusting unit in a tenth embodiment of the present invention, which indicates a variant of the exothermic member 13. In the tenth embodiment, a heat pipe 38 of an extendable shape is employed as the exothermic member 13 used in the eighth embodiment (as shown in Fig. 16) and a heater 39 is mounted on a one end portion of the heat pipe 38.

This arrangement of the exothermic member can simplify the structure of the humidification adjusting unit because it is not necessary to ensure insulation in a region where the multiple hollow fibers 14 are held and humidified. Further, using a general intensity member for the heat pipe 38, the posture of the multiple hollow fibers 14 held to the heat pipe can be enhanced to a higher extent.

Further, by using the heater 39 as a heat source of an endothermic portion of the heat pipe 38, the action of the heat pipe 38 as the exothermic member 13 can be more ensured.

### Brief Description of The Drawings

Fig. 1. A whole structural diagram showing a humidifying unit for an artificially respiratory device according to the first embodiment of the present invention.
Fig. 2. A diagram showing an enlarged essential portion of Fig. 1.
Fig. 3. A perspective view showing the humidifying structural body to be employed in the first embodiment of the present invention.
Fig. 4. A view describing the one connector of the humidifying structural body in the first embodiment of the present invention.
Fig. 5. A perspective view describing the steps of the preparation of the humidifying structural body in the first embodiment of the present invention.
Fig. 6. A perspective view describing the steps of the preparation thereof, following the step of Fig. 5.
Fig. 7. A perspective view describing the steps of the preparation thereof, following the step of Fig. 6.
Fig. 8. A perspective view showing the humidifying structural body according to the second embodiment of the present invention.
Fig. 9. A perspective view showing the humidifying structural body according to the third embodiment of the present invention.
Fig. 10. A perspective view showing the humidifying structural body according to the fourth embodiment of the present invention.
Fig. 11. A perspective view showing the multiple hollow fibers gathering in the tape shape to be employed in the fourth embodiment of the present invention.
Fig. 12. A longitudinally sectional view showing the state in which the multiple hollow fibers gathering in the tape shape is wound.
Fig. 13. A perspective view showing the humidifying structural body according to the fifth embodiment of the present invention.
Fig. 14. A perspective view showing the humidifying structural body according to the sixth embodiment of the present invention.
Fig. 15. A perspective view showing the humidifying structural body according to the seventh embodiment of the present invention.
Fig. 16. A perspective view showing the humidifying structural body according to the eighth embodiment of the present invention.
Fig. 17. A perspective view showing the humidifying structural body according to the ninth embodiment of the present invention.
Fig. 18. A perspective view showing the humidifying structural body according to the tenth embodiment of the present invention.

### Description of Reference Numerals

- 1: Intake tube
- 2: Intake path
- 3: Intake gases
- 4: Humidification adjusting unit
- 5: Short connection tube
- 5a: Tube portion on a one side
- 5b: Tube portion on the other side
- 6: Mounting flange
- 7: Humidifying structural body
- 13: Exothermic member
- 14: Hollow fibers
- 15: Mesh cover tube
- 16: Heating wire
- 17: Insulating member
- 21: One connector
- 22a: Hollow fibers gathering
- 22b: Hollow fibers gathering
- 24: Water bag
- 34: Humidifying unit
- 36: Inner tube
- 37: Outer tube
- 38: Heat pipe
- 39: Heater

## Claims

1. A unit for adjusting humidification, the unit comprising:
an exothermic member (13) having an outer surface;
a plurality of hollow fibers (14) disposed on the outer surface of the exothermic member (13), wherein each hollow fiber in the plurality of hollow fibers (14) includes a water carrying channel defined by a peripheral wall of the hollow fiber for carrying water proximate to the outer surface of the exothermic member (13),
**characterised in that** the peripheral wall of each hollow fiber has a plurality of minute openings that are large enough to allow water vapour to pass therethrough yet small enough to prevent water from passing therethrough so that water carried in the channel of each hollow fiber does not contact the exothermic member.

2. The unit for adjusting humidification of claim 1, wherein the exothermic member (13) is elongated with a length that is greater than its diameter.

3. The unit for adjusting humidification of claim 2, wherein the exothermic member (13) includes a heating wire (16) adapted to generate heat responsive to an electrical current being provided to the heating wire (16).

4. The unit for adjusting humidification of claim 3, wherein the exothermic member (13) further includes an electrical insulating member (17) disposed on the heating wire (16) to electrically insulate the heating wire (16).

5. The unit for adjusting humidification of claim 1, wherein the exothermic member (13) comprises a heat pipe adapted to be heated by a heater disposed at one end thereof.

6. The unit for adjusting humidification of claim 1, wherein the plurality of hollow fibers (14) are disposed on the exothermic member (13) in a spiral manner.

7. The unit for adjusting humidification of claim 1, wherein the plurality of hollow fibers (14) are disposed on the exothermic member (13) such that a longitudinal axis of the plurality of hollow fibers (14) is substantially parallel to a longitudinal axis of the exothermic member (13).

8. The unit for adjusting humidification of claim 1, wherein the plurality of hollow fibers (14) are disposed on the exothermic member (13) such that a longitudinal axis of the plurality of hollow fibers (14) is substantially perpendicular to a longitudinal axis of the exothermic member (13).

9. The unit for adjusting humidification of claim 1, further comprising a water connector (21) provided at a first end of the plurality of hollow fibers (14) to communicate water to an interior of each of the plurality of hollow fibers (14), and wherein a second end of the plurality of hollow fibers (14) is closed.

10. The unit for adjusting humidification of claim 1, further comprising a first water connector (21) provided at a first end of the plurality of hollow fibers (14) and a second water connector (21) provided at a second end of the plurality of hollow fibers (14) opposite the first end, wherein the first and the second fluid connectors (21) are adapted to communicate an interior of each of the plurality of hollow fibers to a water supply system.

11. The unit for adjusting humidification of claim 1, further comprising a perforated covering (15) disposed over the plurality of hollow fibers (14).

12. The unit for adjusting humidification of claim 1, wherein the exothermic member (13) and the plurality of hollow fibers (14) define a humidification element, and further comprising a short connection tube (5) coupled to the humidification element.

13. The unit for adjusting humidification of claim 12, wherein the short connection tube (5) is a generally straight tube having a longitudinal axis and adapted to be connected in an intake circuit, and wherein the short connection tube (5) is configured such that the humidification element is disposed within the tube with a longitudinal axis of the humidification element generally aligned with the longitudinal axis of the tube.

14. The unit for adjusting humidification of claim 12, further comprising a mounting flange (6) to which the humidification element is attached, and wherein the mounting flange (6) is detachably connected to the short connection tube (5).

15. The unit for adjusting humidification of claim 12, wherein the short connection tube (5) is a tube having a first leg (5a) and a second leg (5b), wherein a longitudinal axis of the first leg (5a) intersects a longitudinal axis of the second leg (5b) at a non-zero angle, and wherein the short connection tube is configured such that the humidification element is disposed within the tube with a longitudinal axis of the humidification element generally aligned with a longitudinal axis of one of the first or second legs (5a, 5b) of the tube (5).

16. The unit for adjusting humidification of claim 15, further comprising a second tube (37) disposed about at least a portion of the first leg (5a) or the second leg (5b).

17. The unit for adjusting humidification of claim 15, further comprising a mounting flange (6) to which the humidification element is attached, and wherein the mounting flange (6) is detachably connected to the short connection tube (5).

18. An artificial respiratory system comprising:
(a) source of intake gases adapted to generate a flow of breathing gas (3);
(b) an intake circuit coupled to the source of intake gases so as to deliver the flow of breathing gas to a patient; and
(c) a unit for adjusting humidification disposed in the intake circuit, the unit for adjusting humidification being as defined in any one of claims 1 to 17.

19. The artificial respiratory system of claim 18, wherein the exothermic member (13) and the plurality of hollow fibers (14) define a humidification element, and further comprising a short connection tube (5) coupled to the humidification element and removeably to the intake circuit.

20. The artificial respiratory system of claim 18, further comprising a water delivery system coupled to the plurality of hollow fibers so as to deliver water thereto.

21. The artificial respiratory system of claim 20, wherein the water delivery system includes:
a water source (24);
a water communication conduit (25) coupled to the water source (24) and the plurality of hollow fibers (14);
a water flow monitor (27) associated with the water communication conduit (25) for monitoring a flow of water therethrough; and
an alarm system (33) associated with the flow monitor (27) that provides an alarm responsive to the flow of water in the water communication conduit (25) falling outside a predetermined threshold.

22. The artificial respiratory system of claim 20, wherein the water delivery system includes:
a water source (24);
a water communication conduit (25) coupled to the water source (24) and the plurality of hollow fibers (14); and
a water control system associated with the water source or the water communication conduit (25) for controlling a flow of the water to the plurality of hollow fibers (14).

23. The artificial respiratory system of claim 18, further comprising:
a power source (30);
an electrical connector (28) coupled to the power source (30) and the exothermic member (13) for supplying electrical power (30) from the power source (30) to the exothermic member (13).

24. The artificial respiratory system of claim 23, further comprising a power control system (29) associated with the power source (30) or the electrical connector (28) for controlling the power provided to the exothermic member (13).

25. The artificial respiratory system of claim 18, further comprising a sensor (31) disposed in the intake circuit and adapted to monitor a characteristic of the flow of breathing gas (3) carried by the intake circuit.

26. The artificial respiratory system of claim 25, further comprising an alarm system (33) associated with the sensor (31) that provides an alarm responsive to a parameter monitored by the sensor (31) falling outside a predetermined threshold.

27. A method for the preparation of a unit for adjusting comprising:
providing a plurality of hollow fibers (14), wherein each hollow fiber in the plurality of hollow fibers (14) is defined by a peripheral wall having minute openings large enough to allow water vapour to pass therethrough yet small enough to prevent water from passing therethrough; and
wrapping the plurality of hollow fibers (14) around an exothermic member (13) such that the plurality of hollow fibers (14) are disposed on the outer surface of the exothermic member (13).

28. The method of claim 27, further comprising arranging the plurality of hollow fibers (14) in a sheet, and wherein wrapping the plurality of hollow fibers (14) around the exothermic member (13) includes wrapping the sheet of hollow fibers such that a longitudinal axis of the plurality of hollow fibers is substantially parallel to a longitudinal axis of the exothermic member.

29. The method of claim 27, further comprising arranging the plurality of hollow fibers (14) in a strip, and wherein wrapping the plurality of hollow fibres (14) around the exothermic member (13) includes wrapping the strip of hollow fibers in a spiral around the exothermic member (13).

30. The method of claim 27, wherein wrapping the strip of hollow fibers around the exothermic member (13) includes wrapping the hollow fibers such that a longitudinal axis of the plurality of hollow fibers is substantially perpendicular to a longitudinal axis of the exothermic member.

31. The method of claim 27, further comprising connecting an end portion of the plurality of hollow fibers to a connector which is adapted for selective connection to a water supply system.

32. The method of claim 27, further comprising disposing a perforated covering over the exothermic member and the plurality of hollow fibers.

33. The method of claim 27, wherein the plurality of hollow fibers are wrapped around the exothermic member such that both ends of the plurality of hollow fibers are disposed at a comment end portion of the exothermic member.

## Patentansprüche

1. Einheit zum Einstellen der Befeuchtung, wobei die Einheit folgendes aufweist:
ein exothermes Element (13) mit einer äußeren Oberfläche;
eine Anzahl hohler Fasern (14), die auf der äußeren Oberfläche des exothermen Elements (13) angeordnet ist, wobei jede hohle Faser in der Anzahl der hohlen Fasern (14) einen wasserführenden Kanal aufweist, der durch eine umfangsseitige Wand der hohlen Faser begrenzt ist, um Wasser nahe der äußeren Oberfläche des exothermen Elements (13) zu führen,
**dadurch gekennzeichnet, dass** die umfangsseitige Wand jeder hohlen Faser eine Anzahl von exakten Öffnungen aufweist, die groß genug sind, um es zu ermöglichen, dass Wasserdampf durch sie hindurch tritt, wobei sie jedoch klein genug sind, um zu verhindern, dass Wasser durch sie hindurch tritt, so dass Wasser, das im Kanal jeder hohlen Faser geführt wird, nicht mit dem exothermen Element in Berührung kommt.

2. Einheit zum Einstellen der Befeuchtung nach Anspruch 1, wobei das exotherme Element (13) langgestreckt ist, so dass seine Länge größer ist als sein Durchmesser.

3. Einheit zum Einstellen der Befeuchtung nach Anspruch 2, wobei das exotherme Element (13) einen Heizdraht (16) aufweist, der dafür ausgelegt ist, als Antwort auf einen elektrischen Strom, der dem Heizdraht (16) zugeführt wird, Wärme zu erzeugen.

4. Einheit zum Einstellen der Befeuchtung nach Anspruch 3, wobei das exotherme Element (13) des Weiteren ein elektrisches Isolationselement (17) aufweist, das auf dem Heizdraht (16) angeordnet ist, um den Heizdraht (16) elektrisch zu isolieren.

5. Einheit zum Einstellen der Befeuchtung nach Anspruch 1, wobei das exotherme Element (13) ein Heizrohr aufweist, das dafür ausgelegt ist, durch eine Heizvorrichtung aufgeheizt zu werden, welche an einem Ende desselben angeordnet ist.

6. Einheit zum Einstellen der Befeuchtung nach Anspruch 1, wobei die Anzahl an hohlen Fasern (14) auf dem exothermen Element (13) spiralförmig angeordnet ist.

7. Einheit zum Einstellen der Befeuchtung nach Anspruch 1, wobei die Anzahl an hohlen Fasern (14) auf dem exothermen Element (13) so angeordnet ist, dass die Longitudinalachse der Anzahl an hohlen Fasern (14) im Wesentlichen parallel zur Longitudinalachse des exothermen Elements (13) ist.

8. Einheit zum Einstellen der Befeuchtung nach Anspruch 1, wobei die Anzahl an hohlen Fasern (14) so auf dem exothermen Element (13) angeordnet ist, dass die Longitudinalachse der Anzahl an hohlen Fasern (14) im Wesentlichen senkrecht zur Longitudinalachse des exothermen Elements (13) ist.

9. Einheit zum Einstellen der Befeuchtung nach Anspruch 1, weiter aufweisend einen Wasseranschluss (21), welcher an einem ersten Ende der Anzahl an hohlen Fasern (14) vorhanden ist, um Wasser in das Innere jeder der Anzahl an hohlen Fasern (14) zu befördern, wobei ein zweites Ende der Anzahl an hohlen Fasern (14) geschlossen ist.

10. Einheit zum Einstellen der Befeuchtung nach Anspruch 1, weiter aufweisend einen ersten Wasseranschluss (21), weicher an einem ersten Ende der Anzahl an hohlen Fasern (14) vorhanden ist, und einen zweiten Wasseranschluss (21), welcher an einem zweiten Ende der Anzahl an hohlen Fasern (14) gegenüber dem ersten Ende vorhanden ist, wobei der erste und der zweite Flüssigkeitsanschluss (21) dafür ausgelegt sind, das Innere jeder der Anzahl an hohlen Fasern mit einem Wasserzufuhrsystem zu koppeln.

11. Einheit zum Einstellen der Befeuchtung nach Anspruch 1, weiter aufweisend eine perforierte Abdeckung (15), welche über der Anzahl an hohlen Fasern (14) angeordnet ist.

12. Einheit zum Einstellen der Befeuchtung nach Anspruch 1, wobei das exotherme Element (13) und die Anzahl an hohlen Fasern (14) ein Befeuchtungselement definieren, weiter aufweisend eine kurze Verbindungsröhre (5), die an das Befeuchtungselement gekoppelt ist.

13. Einheit zum Einstellen der Befeuchtung nach Anspruch 12, wobei die kurze Verbindungsröhre (5) eine im Wesentlichen gerade Röhre ist, welche eine Longitudinalachse aufweist und dafür ausgelegt ist, mit einer Einlassleitung verbunden zu werden, wobei die kurze Verbindungsröhre (5) so konfiguriert ist, dass das Befeuchtungselement so innerhalb der Röhre angeordnet ist, dass die Longitudinalachse des Befeuchtungselements im Wesentlichen mit der Longitudinalachse der Röhre ausgerichtet ist.

14. Einheit zum Einstellen der Befeuchtung nach Anspruch 12, weiter aufweisend einen Anbringungsflansch (6), an dem das Befeuchtungselement angebracht ist, wobei der Anbringungsflansch (6) lösbar mit der kurzen Verbindungsröhre (5) verbunden ist.

15. Einheit zum Einstellen der Befeuchtung nach Anspruch 12, wobei die kurze Verbindungsröhre (5) eine Röhre ist, welche ein erstes Bein (5a) und ein zweites Bein (5b) aufweist, wobei die Longitudinalachse des ersten Beins (5a) die Longitudinalachse des zweiten Beins (5b) unter einem Winkel ungleich Null schneidet und wobei die kurze Verbindungsröhre so korifiguriert ist, dass das Befeuchtungselement innerhalb der Röhre so angeordnet ist, dass die Longitudinalachse des Befeuchtungselements im Wesentlichen mit der Longitudinalachse eines der beiden Beine (5a, 5b) der Röhre (5) ausgerichtet ist.

16. Einheit zum Einstellen der Befeuchtung nach Anspruch 15, weiter aufweisend eine zweite Röhre (37), welche zumindest um einen Teil des ersten Beins (5a) oder des zweiten Beins (5b) angeordnet ist.

17. Einheit zum Einstellen der Befeuchtung nach Anspruch 15, weiter aufweisend einen Anbringungsflansch (6), an dem das Befeuchtungselement angebracht ist, wobei der Anbringungsflansch (6) lösbar mit der kurzen Verbindungsröhre (5) verbunden ist.

18. Künstliches Atmungssystem, aufweisend:
(a) eine Quelle von Ansauggasen, die dafür ausgelegt ist, eine Strömung von Atemgas (3) zu erzeugen;
(b) eine Einlassleitung, welche mit der Quelle an Ansauggasen gekoppelt ist, um die Strömung der Atemgase einem Patienten zuzuführen; und
(c) eine Einheit zum Einstellen der Befeuchtung, welche in der Einlassleitung angeordnet ist, wobei die Einheit zum Einstellen der Befeuchtung gemäß einem der Ansprüche 1 bis 17 ausgebildet ist.

19. Künstliches Atmungssystem nach Anspruch 18, wobei das exotherme Element (13) und die Anzahl an hohlen Fasern (14) ein Befeuchtungselement befinden, weiter aufweisend eine kurze Verbindungsröhre (5), die an das Befeuchtungselement und entfernbar an die Einlassleitung gekoppelt ist.

20. Künstliches Atmungssystem nach Anspruch 18, weiter aufweisend ein Wasserabgabesystem, das an die Anzahl an hohlen Fasern gekoppelt ist, um diesen Wasser zuzuführen.

21. Künstliches Atmungssystem nach Anspruch 20, wobei das Wasserzufuhrsystem folgendes aufweist:
eine Wasserquelle (24);
eine Wasseranschlussleitung (25), die mit der Wasserquelle (24) und der Anzahl an hohlen Fasern (14) gekoppelt ist;
ein Wasserströmungsgerät (27), das der Wasseranschlussleitung (25) zugeordnet ist, um die Strömung von Wasser durch diese zu überwachen; und
ein Alarmsystem (33), das dem Strömungsüberwachungsgerät (27) zugeordnet ist und als Antwort darauf, dass die Strömung von Wasser in der Wasseranschlussleitung aus einem vorgegebenen Schwellbereich herausfällt, einen Alarm ausgibt.

22. Künstliches Atmungssystem nach Anspruch 20, wobei das Wasserzufuhrsystem folgendes aufweist:
eine Wasserquelle (24);
eine Wasseranschlussleitung (25), die mit der Wasserquelle (24) und der Anzahl an hohlen Fasern (14) gekoppelt ist; und
ein Wasserregelsystem, das der Wasserquelle oder der Wasseranschlussleitung (25) zugeordnet ist, um die Strömung des Wassers zur Anzahl der hohlen Fasern (14) zu regeln.

23. Künstliches Atmungssystem nach Anspruch 18, weiter aufweisend:
eine Energiequelle (30);
einen elektrischen Anschluss (28), der mit der Energiequelle (30) und dem exothermen Element (13) gekoppelt ist, um elektrische Leistung (30) von der Energiequelle (30) dem exothermen Element (13) zuzuführen.

24. Künstliches Atmungssystem nach Anspruch 23, weiter aufweisend ein Leistungsregelungssystem (29), das der Energiequelle (30) oder dem elektrischen Anschluss (28) zugeordnet ist, um die dem exothermen Element (13) zugeführte Leistung zu regeln.

25. Künstliches Atmungssystem nach Anspruch 18, weiter aufweisend einen Sensor (31), der in der Einlassschaltung angeordnet ist und dafür ausgelegt ist, eine charakteristische Eigenschaft der Strömung des Atemgases (3), welches durch die Einlassleitung befördert wird, zu überwachen.

26. Künstliches Atmungssystem nach Anspruch 25, weiter aufweisend ein Alarmsystem (33), das dem Sensor (31) zugeordnet ist und einen Alarm als Antwort darauf, das ein vom Sensor (31) überwachter Parameter außerhalb eines vorgegebenen Schwellwertes liegt, ausgibt.

27. Verfahren zur Herstellung einer Einheit zum Einstellen der Befeuchtung, aufweisend:
Bereitstellen einer Anzahl an hohlen Fasern (14), wobei jede hohle Faser der Anzahl an hohlen Fasern (14) durch eine umfangsseitige Wand begrenzt ist, welche exakte Öffnungen aufweist, die groß genug sind, um es zu ermöglichen, dass Wasserdampf durch sie hindurchtritt, jedoch klein genug, um Wasser am Durchtreten zu hindern; und
Wickeln der Anzahl an hohlen Fasern (14) um ein exothermes Element (13), so dass die Anzahl an hohlen Fasern (14) auf der äußeren Oberfläche des exothermen Elements (13) angeordnet ist.

28. Verfahren nach Anspruch 27, weiter aufweisend das Anordnen der Anzahl an hohlen Fasern (14) in einer Schicht, wobei das Wickeln der Anzahl an hohlen Fasern (14) um das exotherme Element (13) das Wickeln der Schicht an hohlen Fasern auf eine Weise umfasst, dass die Longitudinalachse der Anzahl an hohlen Fasern im Wesentlichen parallel zur Longitudinalachse des exothermen Elements ist.

29. Verfahren nach Anspruch 27, weiter aufweisend das Anordnen der Anzahl an hohlen Fasern (14) ein einem Streifen, wobei das Wickeln der Anzahl an hohlen Fasern (14) um das exotherme Element (13) das spiralförmige Wickeln des Streifens aus hohlen Fasern um das exotherme Element (13) beinhaltet.

30. Verfahren nach Anspruch 27, wobei das Wickeln des Streifens aus hohlen Fasern um das exotherme Element (13) das Wickeln der hohlen Fasern auf eine Weise umfasst, dass die Longitudinalachse der Anzahl an hohlen Fasern im Wesentlichen senkrecht zur Longitudinalachse des exothermen Elements ist.

31. Verfahren nach Anspruch 27, weiter aufweisend das Verbinden eines Endabschnitts der Anzahl an hohlen Fasern mit einem Anschlusselement, das für eine selektive Verbindung mit einem Wasserzufuhrsystem ausgelegt ist.

32. Verfahren nach Anspruch 27, weiter aufweisend das Anordnen einer perforierten Abdeckung über dem exothermen Element und der Anzahl an hohlen Fasern.

33. Verfahren nach Anspruch 27, wobei die Anzahl an hohlen Fasern so um das exotherme Element gewickelt ist, dass beide Enden der Anzahl an holen Fasern an einem vermerkten Endabschnitt des exothermen Elements angeordnet sind.

## Revendications

1. Une unité de réglage d'humidification, l'unité comprenant :
un élément exothermique (13) comprenant une surface externe ;
une pluralité de fibres creuses (14) disposées sur la surface externe de l'élément exothermique (13), chaque fibre creuse de la pluralité de fibres creuses (14) comprenant une canalisation de transport d'eau définie par une paroi périphérique de la fibre creuse pour transporter de l'eau à proximité de la surface externe de l'élément exothermique (13),
**caractérisée en ce que** la paroi périphérique de chaque fibre creuse présente une pluralité d'ouvertures très petites qui sont suffisamment grandes pour permettre à la vapeur d'eau de passer à travers elles et cependant suffisamment petites pour empêcher l'eau de passer à travers elles de sorte que l'eau transportée dans la canalisation de chaque fibre creuse n'est pas en contact avec l'élément exothermique.

2. L'unité de réglage d'humidification de la revendication 1, dans laquelle l'élément exothermique (13) est allongé en présentant une longueur qui est plus grande que son diamètre.

3. L'unité de réglage d'humidification de la revendication 2, dans laquelle l'élément exothermique (13) comprend un fil de chauffage (16) adapté pour produire de la chaleur en réponse à un courant électrique fourni au fil de chauffage (16).

4. L'unité de réglage d'humidification de la revendication 3, dans laquelle l'élément exothermique (13) comprend en outre un élément isolant de l'électricité (17) disposé sur le fil de chauffage (16) pour isoler électriquement le fil de chauffage (16).

5. L'unité de réglage d'humidification de la revendication 1, dans laquelle l'élément exothermique (13) comprend un tuyau de chaleur adapté pour être chauffé par un organe de chauffage disposé à une extrémité de celui-ci.

6. L'unité de réglage d'humidification de la revendication 1, dans laquelle les fibres de la pluralité de fibres creuses (14) sont disposées en spirale sur l'élément exothermique (13).

7. L'unité de réglage d'humidification de la revendication 1, dans laquelle les fibres de la pluralité de fibres creuses (14) sont disposées sur l'élément exothermique (13) de telle manière qu'un axe longitudinal de la pluralité de fibres creuses (14) soit sensiblement parallèle à un axe longitudinal de l'élément exothermique (13).

8. L'unité de réglage d'humidification de la revendication 1, dans laquelle les fibres de la pluralité de fibres creuses (14) sont disposées sur l'élément exothermique (13) de telle manière qu'un axe longitudinal de la pluralité de fibres creuses (14) soit sensiblement perpendiculaire à un axe longitudinal de l'élément exothermique (13).

9. L'unité de réglage d'humidification de la revendication 1, comprenant en outre un connecteur d'eau (21) prévu à une première extrémité de la pluralité de fibres creuses (14) pour faire communiquer de l'eau vers l'intérieur de chacune des fibres de la pluralité de fibres creuses (14), une seconde extrémité de la pluralité de fibres creuses (14) étant fermée.

10. L'unité de réglage d'humidification de la revendication 1, comprenant en outre un premier connecteur d'eau (21) prévu à une première extrémité de la pluralité de fibres creuses (14) et un second connecteur d'eau (21) prévu à une seconde extrémité de la pluralité de fibres creuses (14) opposée à la première extrémité, les premier et second connecteurs de fluide (14) étant adaptés pour faire communiquer une partie interne de chaque fibre de la pluralité de fibres creuses à un système d'alimentation en eau.

11. L'unité de réglage d'humidification de la revendication 1, comprenant en outre un recouvrement perforé (15) disposé sur la pluralité de fibres creuses (14).

12. L'unité de réglage d'humidification de la revendication 1, dans laquelle l'élément exothermique (13) et la pluralité de fibres creuses (14) définissent un élément d'humidification, et comprenant en outre un court tube de liaison (5) couplé à l'élément d'identification.

13. L'unité de réglage d'humidification de la revendication 12, dans laquelle le court tube de liaison (5) est un tube généralement rectiligne présentant un axe longitudinal et adapté pour être relié à un circuit d'entrée, et dans lequel le court tube de liaison (5) est configuré de telle manière que l'élément d'identification soit disposé à l'intérieur du tube avec un axe longitudinal de l'élément d'identification qui est de manière générale aligné avec l'axe longitudinal du tube.

14. L'unité de réglage d'humidification de la revendication 12, comprenant en outre une bride de montage (6) à laquelle est fixé l'élément d'identification, et dans laquelle la bride de montage (6) est reliée de manière amovible au court tube de liaison (5).

15. L'unité de réglage d'humidification de la revendication 12, dans laquelle le court tube de liaison (5) est un tube comprenant une première branche (5a) et une seconde branche (5b), dans laquelle un axe longitudinal de la première branche (5a) coupe un axe longitudinal de la seconde branche (5b) selon un angle non nul, et dans laquelle le court tube de liaison est configuré de telle manière que l'élément d'humidification soit disposé à l'intérieur du tube avec un axe longitudinal de l'élément d'humidification qui est de manière générale aligné avec un axe longitudinal d'une des première ou seconde branches (5a, 5b) du tube (5).

16. L'unité de réglage d'humidification de la revendication 15, comprenant en outre un second tube (37) disposé autour d'au moins une partie de la première branche (5a) ou de la seconde branche (5b).

17. L'unité de réglage d'humidification de la revendication 15, comprenant en outre une bride de montage (6) à laquelle est fixé l'élément d'humidification, et dans laquelle la bride de montage (6) est reliée de manière amovible au court tube de liaison (5).

18. Un système de respiration artificielle comprenant :
(a) une source de gaz d'entrée adaptée pour produire un flux de gaz respiratoire (3) ;
(b) un circuit d'entrée couplé à la source de gaz d'entrée de manière à fournir à un patient le flux de gaz respiratoire ; et
(c) une unité de réglage d'humidification disposée dans le circuit d'entrée, l'unité de réglage d'humidification étant telle que définie dans l'une quelconque des revendications 1 à 17.

19. Le système de respiration artificielle de la revendication 18, dans lequel l'élément exothermique (13) et la pluralité de fibres creuses (14) définissent un élément d'humidification, et comprenant en outre un court tube de liaison (5) couplé à l'élément d'humidification et, de manière amovible, au circuit d'entrée.

20. Le système de respiration artificielle de la revendication 18, comprenant en outre un système d'amenée d'eau couplé à la pluralité de fibres creuses de manière à y amener de l'eau.

21. Le système de respiration artificielle de la revendication 20, dans lequel le système d'amenée d'eau comprend :
une source d'eau (24) ;
un conduit de communication d'eau (25) couplé à la source d'eau (24) et à la pluralité de fibres creuses (14);
un organe de contrôle de flux d'eau (27) associé au conduit de communication d'eau (25) pour contrôler un flux d'eau à travers lui ; et
un système d'alarme (33) associé à l'organe de contrôle de flux (27) qui fournit une alarme en réponse au fait que le flux d'eau dans le conduit de communication d'eau (25) tombe à l'extérieur d'un seuil prédéterminé.

22. Le système de respiration artificielle de la revendication 20, dans lequel le système d'amenée d'eau comprend:
une source d'eau (24) ;
un conduit de communication d'eau (25) couplé à la source d'eau (24) et à la pluralité de fibres creuses (14) ; et
un système de commande d'eau associé à la source d'eau ou au conduit de communication d'eau (25) pour commander un flux de l'eau vers la pluralité de fibres creuses (14).

23. Le système de respiration artificielle de la revendication 18, comprenant en outre :
une source d'énergie électrique (30) ;
un connecteur électrique (28) couplé à la source d'énergie électrique (30) et à l'élément exothermique (13) pour amener de l'énergie électrique (30) depuis la source d'énergie électrique (30) jusqu'à l'élément exothermique (13).

24. Le système de respiration artificielle de la revendication 23, comprenant en outre un système de commande d'énergie électrique (29) associé à la source d'énergie électrique (30) ou au connecteur électrique (28) pour commander l'énergie électrique amenée à l'élément exothermique (13).

25. Le système de respiration artificielle de la revendication 18, comprenant en outre un capteur (31) disposé dans le circuit d'entrée et adapté à contrôler une caractéristique du flux de gaz respiratoire (3) transporté par le circuit d'entrée.

26. Le système de respiration artificielle de la revendication 25, comprenant en outre un système d'alarme (33) associé au capteur (31) et fournissant une alarme en réponse au fait qu'un paramètre contrôlé par le capteur (31) tombe à l'extérieur d'un seuil prédéterminé.

27. Un procédé pour la préparation d'une unité de réglage d'humidification comprenant les étapes consistant à :
créer une pluralité de fibres creuses (14), chaque fibre creuse de la pluralité de fibres creuses (14) étant définie par une paroi périphérique présentant de très petites ouvertures suffisamment grandes pour permettre à la vapeur d'eau de passer à travers elles et cependant suffisamment petites pour empêcher l'eau de passer à travers elles ; et
envelopper la pluralité de fibres creuses (14) autour d'un élément exothermique (13) de telle manière que la pluralité de fibres creuses (14) soit disposée sur la surface externe de l'élément exothermique (13).

28. Le procédé de la revendication 27, comprenant en outre le fait de disposer la pluralité de fibres creuses (14) en une nappe, et dans lequel l'étape consistant à envelopper la pluralité de fibres creuses (14) autour de l'élément exothermique (13) comprend le fait d'envelopper la nappe de fibres creuses de telle manière qu'un axe longitudinal de la pluralité de fibres creuses soit sensiblement parallèle à un axe longitudinal de l'élément exothermique.

29. Le procédé de la revendication 27, comprenant en outre le fait de disposer la pluralité de fibres creuses (14) en une bande, et dans lequel l'étape consistant à envelopper la pluralité de fibres creuses (14) autour de l'élément exothermique (13) comprend le fait d'envelopper en spirale la bande de fibres creuses autour de l'élément exothermique (13).

30. Le procédé de la revendication 27, dans lequel l'étape consistant à envelopper la bande de fibres creuses autour de l'élément exothermique (13) comprend le fait d'envelopper les fibres creuses de telle manière qu'un axe longitudinal de la pluralité de fibres creuses soit sensiblement perpendiculaire à un axe longitudinal de l'élément exothermique.

31. Le procédé de la revendication 27, comprenant en outre le fait de relier une partie d'extrémité de la pluralité de fibres creuses à un connecteur qui est adapté pour une liaison sélective à un système d'amenée d'eau.

32. Le procédé de la revendication 27, comprenant en outre le fait de disposer un recouvrement perforé sur l'élément exothermique et la pluralité de fibres creuses.

33. Le procédé de la revendication 27, dans lequel les fibres de la pluralité de fibres creuses sont enveloppées autour de l'élément exothermique de telle manière que les deux extrémités de la pluralité de fibres creuses soient disposées à une partie d'extrémité de commentaire de l'élément exothermique.
